(19) Europäisches Patentamt
European Patent Office
Office européen des brevets

(11) EP 0 800 816 B1

(12) **EUROPÄISCHE PATENTSCHRIFT**

(45) Veröffentlichungstag und Bekanntmachung des Hinweises auf die Patenterteilung:
**03.09.2008 Patentblatt 2008/36**

(51) Int Cl.:
***A61K 8/37*** *(2006.01)* ***A61K 8/49*** *(2006.01)*
***A61Q 17/04*** *(2006.01)*

(21) Anmeldenummer: **97105493.7**

(22) Anmeldetag: **03.04.1997**

(54) **Kosmetische und dermatologische Lichtschutzformulierungen mit einem Gehalt an Triazinderivaten und Dialkylestern von alpha,beta-Hdroxycarbon-sàuren**

Cosmetic and dermatological photoprotective compositions containing a triazine derivative and dialkyl esters of alpha, beta-hydroxycarboxylic acids

Compositions cosmétiques et dermatologiques de protection solaire contenant un dérivé de triazine et des esters dialkyliques d'acides alpha, bèta-hydroxycarboxyliques

(84) Benannte Vertragsstaaten:
**AT BE CH DE ES FR GB IT LI NL**

(30) Priorität: **17.04.1996 DE 19615038**

(43) Veröffentlichungstag der Anmeldung:
**15.10.1997 Patentblatt 1997/42**

(73) Patentinhaber: **Beiersdorf Aktiengesellschaft**
**20245 Hamburg (DE)**

(72) Erfinder:
- **Gers-Barlag, Dr.**
  **25495 Kummerfeld (DE)**
- **Kröpke, Rainer**
  **22527 Hamburg (DE)**

(56) Entgegenhaltungen:
**EP-A- 0 635 260** **US-A- 5 302 376**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 0 800 816 B1

## Beschreibung

Die vorliegende Erfindung betrifft kosmetische und dermatologische Lichtschutzzubereitungen, insbesondere hautpflegende kosmetische und dermatologische Lichtschutzzubereitungen.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutze gegen UVB-Strahlung sind zahlreiche Verbindungen bekannt, bei denen es sich zumeist um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, den sogenannten UVA-Bereich, ist es wichtig, Fitersubstanzen zur Verfügung zu haben, da auch dessen Strahlen Schäden hervorrufen können. So ist erwiesen, daß UVA-Strahlung zu einer Schädigung der elastischen und kollagenen Fasern des Bindegewebes führt, was die Haut vorzeitig altern läßt, und daß sie als Ursache zahlreicher phototoxischer und photoallergischer Reaktionen zu sehen ist. Der schädigende Einfluß der UVB-Strahlung kann durch UVA-Strahlung verstärkt werden.

Die UV-Strahlung kann aber auch zu photochemischen Reaktionen führen, wobei dann die photochemischen Reaktionsprodukte in den Hautmetabolismus eingreifen.

Vorwiegend handelt es sich bei solchen photochemischen Reaktionsprodukten um radikalische Verbindungen, z.B. Hydroxylradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele Andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Ferner zählt UV-Strahlung zur ionisierenden Strahlung. Es besteht also das Risiko, daß auch ionische Spezies bei UV-Exposition entstehen, welche dann ihrerseits oxidativ in die biochemischen Prozesse einzugreifen vermögen.

Ein vorteilhafter UVB-Filter ist der 4,4',4''-(1,3,5-Triazin-2,4,6-triyltriimino)-tris-benzoesäure-tris(2-ethylhexylester), synonym: 2,4,6-Tris-[anilino-(p-carbo-2'-ethyl-1'-hexyloxy)]-1,3,5-triazin.

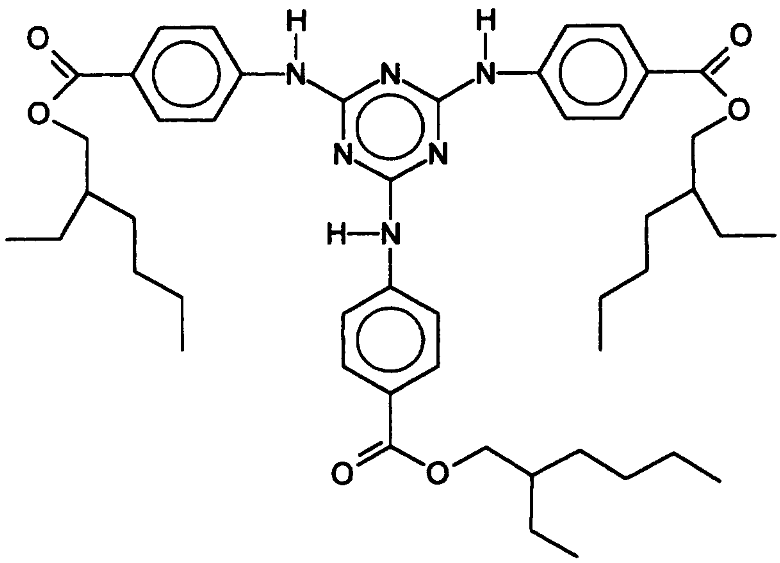

Diese UVB-Filtersubstanz wird von der BASF Aktiengesellschaft unter der Warenbezeichnung UVINUL® T 150 vertrieben und zeichnet sich durch gute UV-Absorptionseigenschaften aus.

Der Hauptnachteil dieses UVB-Filters ist die schlechte Löslichkeit in Lipiden. Bekannte Lösungsmittel für diesen UVB-Filter können maximal ca. 15 Gew.-% dieses Filters lösen, entsprechend etwa 1 - 1,5 Gew.-% gelöster, und damit aktiver, UV-Filtersubstanz.

Es war indes überraschend und für den Fachmann nicht vorauszusehen, daß lichtschutzwirksame Wirkstoffkombinationen aus 4,4',4''-(1,3,5-Triazin-2,4,6-triyltriimino)-tris-benzoesäure-tris(2-ethylhexylester) und einem oder mehreren Dialkylestern von α,β-Dihydroxycarbonsäuren, wobei diese α,β-Dihydroxycarbonsäurediester die allgemeine Formel

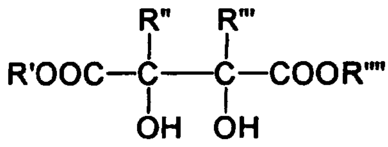

besitzen,

(a) wobei R'' und R''' unabhängig voneinander gewählt werden aus der Gruppe

(a1) H-,
(a2) verzweigtes oder unverzweigtes $C_{1-25}$-Alkyl-,
(a3) mit einer oder mehreren Carboxylgruppen und/oder Hydroxygruppen und/oder Aldehydgruppen und/oder Oxogruppen (Ketogruppen) substituiertes verzweigtes oder unverzweigtes $C_{1-25}$-Alkyl-

oder
(b) wobei das α-Kohlenstoffatom der α-Hydroxycarbonsäure mit R' und R'' zusammen eine

(b1) unsubstituierte Cycloalkylgruppe mit 3 bis 7 Ringatomen oder eine
(b2) mit einer oder mehreren Carboxylgruppen und/oder Hydroxygruppen und/oder Oxogruppen (Ketogruppen) und/oder verzweigten und/oder unverzweigten $C_{1-25}$-Alkylgruppen substituierte Cycloalkylgruppe mit 3 bis 7 Ringatomen

ausbildet und
wobei R' und R'''' unabhängig voneinander gewählt werden aus der Gruppe verzweigtes oder unverzweigtes $C_{1-25}$-Alkyl-, den Nachteilen des Standes der Technik abhelfen

Erfindungsgemäß ist insbesondere auch die Verwendung von Dialkylestern von α,β-Dihydroxycarbonsäuren, wobei diese α,β-Dihydroxycarbonsäurediester die allgemeine Formel

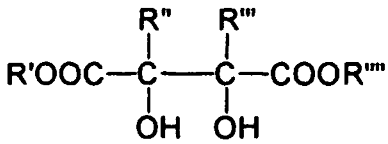

besitzen,

(a) wobei R'' und R'''unabhängig voneinander gewählt werden aus der Gruppe

(a1) H-,
(a2) verzweigtes oder unverzweigtes $C_{1-25}$-Alkyl-,
(a3) mit einer oder mehreren Carboxylgruppen und/oder Hydroxygruppen und/oder Aldehydgruppen und/oder Oxogruppen (Ketogruppen) substituiertes verzweigtes oder unverzweigtes $C_{1-25}$-Alkyl-

oder
(b) wobei das α-Kohlenstoffatom der α-Hydroxycarbonsäure mit R' und R'' zusammen eine

(b1) unsubstituierte Cycloalkylgruppe mit 3 bis 7 Ringatomen oder eine
(b2) mit einer oder mehreren Carboxylgruppen und/oder Hydroxygruppen und/oder Oxogruppen (Ketogruppen) und/oder verzweigten und/oder unverzweigten $C_{1-25}$-Alkylgruppen substituierte Cycloalkylgruppe mit 3 bis 7 Ringatomen

ausbildet und
wobei R' und R'''' unabhängig voneinander gewählt werden aus der Gruppe verzweigtes oder unverzweigtes $C_{1-25}$-Alkyl-, als Lösungsmittel, Lösungsvermittler oder Solubilisator für 4,4',4''-(1,3,5-Triazin-2,4,6-triyltriimino)-tris-benzoesäure-tris(2-ethylhexylester), insbesondere für die Verwendung in Lichtschutzmitteln.

Voraussetzung für die Verwendbarkeit der erfindungsgemäßen Wirkstoffkombinationen für die erfindungsgemäßen Zwecke ist natürlich die kosmetische bzw. dermatologische Unbedenklichkeit der zugrundeliegenden Substanzen.

Es ist erfindungsgemäß möglich die Einsatzmengen von 4,4',4''-(1,3,5-Triazin-2,4,6-triyltriimino)-tris-benzoesäure-tris(2-ethylhexylester) in kosmetischen oder dermatologischen Zubereitungen gegenüber dem Stande der Technik zu verdoppeln.

Zwar ist aus der Europäischen Patentanmeldung 685 226 bekannt, bestimmte Diester und Triester von α-Hydroxycarbonsäuren in Kombination mit 4,4',4''-(1,3,5-Triazin-2,4,6-triyltriimino)-tris-benzoesäure-tris(2-ethylhexylester) in kosmetischn Lichtschutzzubereitungen zu verwenden. Der Fachmann wurde aber durch diese Schrift nicht auf den Weg zur vorliegenden Erfindung, welche α,β-Dihydroxycarbonsäurediester betrifft, geführt.

Ferner war erstaunlich, daß durch Zugabe erfindungsgemäßer α,β-Dihydroxycarbonsäurediester eine Stabilisierung von Lösungen des 4,4',4''-(1,3,5-Triazin-2,4,6-triyltriimino)-tris-benzoesäure-tris(2-ethylhexylester) bewirkt wird, da die letztere Substanz nicht nur schlechte Löslichkeit aufweist, sondern auch aus seiner Lösung leicht wieder auskristallisiert. Erfindungsgemäß ist daher auch ein Verfahren zur Stabilisierung von Lösungen des 4,4',4''-(1,3,5-Triazin-2,4,6-triyltriimino)-tris-benzoesäure-tris(2-ethylhexylester), dadurch gekennzeichnet, daß solchen Lösungen ein wirksamer Gehalt an erfindungsgemäßen α,β-Dihydroxycarbonsäurediestern zugesetzt wird.

Besonders bevorzugt sind die Kombinationen, welche als α,β-Dihydroxycarbonsäurediester
Ester der Weinsteinsäure enthalten, insbesondere $C_{10-18}$-Alkylester der Weinsteinsäure, bevorzugt $C_{12-13}$-Alkylester der Weinsteinsäure enthalten.

Solche $C_{12-13}$-Alkylester der Weinsteinsäure sind beispielsweise erhältlich unter der Produktbezeichnung "COSMACOL ETI" der Gesellschaft EniChem Augusta Industriale.

Die Gesamtmenge an 4,4',4''-(1,3,5-Triazin-2,4,6-triyltriimino)-tris-benzoesäure-tris(2-ethylhexylester) in den fertigen kosmetischen oder dermatologischen Zubereitungen wird vorteilhaft aus dem Bereich von 0,1 - 10,0 Gew.-%, bevorzugt 0,5 - 6,0 Gew.-% gewählt, bezogen auf das Gesamtgewicht der Zubereitungen.

Die Gesamtmenge an einem oder mehreren erfindungsgemäßen α,β-Dihydroxycarbonsäurediestern in den fertigen kosmetischen oder dermatologischen Zubereitungen wird vorteilhaft aus dem Bereich von 0,1 - 25,0 Gew.-%, bevorzugt 0,5 - 15,0 Gew.-% gewählt, bezogen auf das Gesamtgewicht der Zubereitungen.

Es ist Von Vorteil, Gewichtsverhältnisse von 4,4',4''-(1,3,5-Triazin-2,4,6-triyltriimino)-tris-benzoesäure-tris(2-ethylhexylester) und einem oder mehreren erfindungsgemäßen α,β-Dihydroxycarbonsäurediestern aus dem Bereich von 1 : 10 bis 10 : 1, bevorzugt 1 : 4 bis 4 : 1, zu wählen.

Erfindungsgemäße kosmetische und dermatologische Zubereitungen enthalten vorteilhart außerdem anorganische Pigmente auf Basis von Metalloxiden und/oder anderen in Wasser schwerlöslichen oder unlöslichen Metallverbindungen, insbesondere der Oxide des Titans ($TiO_2$), Zinks (ZnO), Eisens (z.B. $Fe_2O_3$), Zirkoniums ($ZrO_2$), Siliciums ($SiO_2$), Mangans (z.B. MnO), Aluminiums ($Al_2O_3$), Cers (z.B. $Ce_2O_3$), Mischoxiden der entsprechenden Metalle sowie Abmischungen aus solchen Oxiden. Besonders bevorzugt handelt es sich um Pigmente auf der Basis von $TiO_2$.

Es ist besonders vorteilhaft im Sinne der vorliegenden Erfindung, wenngleich nicht zwingend, wenn die anorganischen Pigmente in hydrophober Form vorliegen, d.h., daß sie oberflächlich wasserabweisend behandelt sind. Diese Oberflächenbehandlung kann darin bestehen, daß die Pigmente nach an sich bekannten Verfahren mit einer dünnen hydrophoben Schicht versehen werden.

Eines solcher Verfahren besteht beispielsweise darin, daß die hydrophobe Oberflächenschicht nach einer Rektion gemäß

$$n\ TiO_2 + m\ (RO)_3\,Si\text{-}R' \text{ -> } n\ TiO_2\ (\text{oberfl.})$$

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte $TiO_2$-Pigmente sind beispielsweise unter den Handelsbezeichnungen MT 100 T von der Firma TAYCA, ferner M 160 von der Firma Kemira sowie T 805 von der Firma Degussa erhältlich.

Die erfindungsgemäßen kosmetischen und/oder dermatologischen Lichtschutzformulierungen können wie üblich zusammengesetzt sein und dem kosmetischen und/oder dermatologischen Lichtschutz, ferner zur Behandlung, der

Pflege und der Reinigung der Haut und/oder der Haare und als Schminkprodukt in der dekorativen Kosmetik dienen.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Besonders bevorzugt sind solche kosmetischen und dermatologischen Zubereitungen, die in der Form eines Sonnenschutzmittels vorliegen. Vorteilhaft können diese zusätzlich mindestens einen weiteren UVA-Filter und/oder mindestens einen weiteren UVB-Filter und/oder mindestens ein anorganisches Pigment, bevorzugt ein anorganisches Mikropigment, enthalten.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Furfurylidensorbitol und dessen Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, $ZnSO_4$) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Die Lipidphase kann vorteilhaft gewählt werden aus folgender Substanzgruppe:

- Mineralöle, Mineralwachse
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewährt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteillhaft wird die Ölphase gewählt aus der Gruppe 2-Ethylhexylisostearat, Octyldodecanol, Isotridecylisononanoat, Isoeicosan, 2-Ethylhexylcocoat, $C_{12-15}$-Alkylbenzoat, Capryl-Caprinsäure-triglycerid, Dicaprylylether.

Besonders vorteilhaft sind Mischungen aus $C_{12-15}$-Alkybenzoat und 2-Ethylhexylisostearat, Mischungen aus $C_{12-15}$-Alkybenzoat und Isotridecylisononanoat sowie Mischungen aus $C_{12-15}$-Alkybenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wäßrige Phase der erfindungsgemäßen Zubereitungen enthält gegebenenfalls vorteilhaft

- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewährt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Die kosmetischen oder dermatologischen Lichtschutzzubereitungen enthalten vorteilhaft anorganische Pigmente, insbesondere Mikropigmente, z.B. in Mengen von 0,1 Gew.-% bis 30 Gew.-%, vorzugsweise in Mengen von 0,5 Gew.-% bis 10 Gew.-%, insbesondere aber 1 Gew.-% bis 6 Gew.-%, bezogen auf das Gesamtgewicht der Zubereitungen.

Es ist erfindungsgemäß vorteilhaft, außer den erfindungsgemäßen Kombinationen öllösliche UVA-Filter und/ oder UVB-Filter in der Lipidphase und/oder wasserlösliche UVA-Filter und/oder UVB-Filter in der wäßrigen Phase einzusetzen.

Vorteilhaft können die erfindungsgemäßen Lichtschutzformulierungen weitere Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitungen, um kosmetische Zubereitungen zur Verfügung zu stellen, die die Haut vor dem gesamten Bereich der ultravioletten Strahlung schützen. Sie können auch als Sonnenschutzmittel dienen.

Die weiteren UVB-Filter können öllöslich oder wasserlöslich sein. Vorteilhafte öllösliche UVB-Filtersubstanzen sind z.B.:

- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Zimtsäure, vorzugsweise 4-Methoxyimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;

Vorteilhafte wasserlösliche UVB-Filtersubstanzen sind z.B.:

- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und deren Salze;
- Sulfonsäure-Derivate des 3-Benzylidencamphers, wie z.B. 4-(2-Oxo-3-bornylidenmethyl)benzolsulfonsäure,2-Methyl-5-(2-oxo-3-bornylidenmethyl)sulfonsäure und deren Salze.

Die Liste der genannten weiteren UVB-Filter, die in Kombination mit den erfindungsgemäßen Wirkstoffkombinationen verwendet werden können, soll selbstverständlich nicht limitierend sein.

Es kann auch von Vorteil sein, die erfindungsgemäßen Kombinationen mit weiteren UVA-Filtern zu kombinieren, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch diese Kombinationen bzw. Zubereitungen, die diese Kombinationen enthalten, sind Gegenstand der Erfindung. Es können die für die UVB-Kombination verwendeten Mengen eingesetzt werden.

Ferner ist vorteilhaft, die erfindungsgemäßen Wirkstoffkombinationen mit weiteren UVA-und/oder UVB-Filtern zu kombinieren.

Es ist auch besonders vorteilhaft, die erfindungsgemäßen Wirkstoffkombinationen mit Salicylsäurederivaten zu kombinieren, von denen einige Vertreter bekannt sind, welche ebenfalls UV-Strahlung absorbieren können. Zu gebräuchlichen UV-Filtern gehören

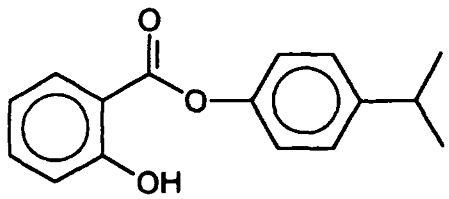

**(4-Isopropylbenzylsalicylat),**

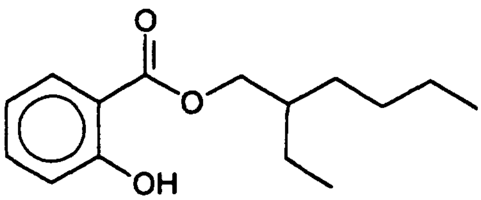

**(2-Ethylhexylsalicylat, Octylsalicylat),**

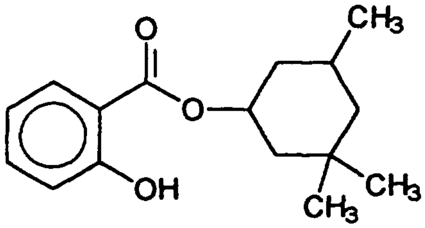

(Homomenthylsalicylat).

Gegenstand der Erfindung ist auch ein Verfahren zur Herstellung der erfindungsgemäßen kosmetischen und/ oder dermatologischen Lichtschutzzubereitungen, das dadurch gekennzeichnet ist, daß man in an sich bekannter Weise den 4,4',4''-(1,3,5-Triazin-2,4,6-triyltriimino)-tris-benzoesäure-tris(2-ethylhexylester) in einem oder mehreren α,β-Dihydroxycarbonsäurediestern oder einer Ölphase mit einem Gehalt an α,β-Dihydroxycarbonsäurediestern bei gleichmäßigem Rühren und gegebenenfalls unter Erwärmen suspendiert und gewünschtenfalls homogenisiert, gegebenenfalls mit weiteren Lipidkomponenten und gegebenenfalls mit einem oder mehreren Emulgatoren vereinigt, hernach die Ölphase mit der wäßrigen Phase, in welche gegebenenfalls ein Verdickungsmittel eingearbeitet worden ist, und welche vorzugsweise etwa die gleiche Temperatur besitzt wie die Ölphase, vermischt, gewünschtenfalls homogenisiert und auf Raumtemperatur abkühlen läßt. Nach Abkühlen auf Raumtemperatur kann, insbesondere, wenn noch flüchtige Bestandteile eingearbeitet werden sollen, nochmaliges Homogenisieren erfolgen.
Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

## Beispiel 1

| O/W-Emulsion | |
|---|---|
| | Gew.-% |
| Stearinsäure | 3.50 |
| Glycerin | 3.00 |
| Cetearyl Alkohol | 0.50 |
| Konservierungsmittel | q.s. |
| Parfum | q.s. |
| Caprylyl Ether | 8.00 |
| Uvinul T150 | 5.00 |
| Cosmacol ETI | 12.0 |
| Natriumhydroxid (45%ig) | 0.33 |
| Carbomer | 0.20 |
| Wasser, demin. | ad 100.0 |

## Beispiel 2

| W/O-Emulsion | |
|---|---|
| | Gew.-% |
| Arlacel 989 | 5.50 |

(fortgesetzt)

| W/O-Emulsion | |
|---|---|
| | Gew.-% |
| Butylenglykol | 5.00 |
| Konservierungsmittel | q.s. |
| Parfum | q.s. |
| Cosmacol ETI | 12.00 |
| Uvinul T150 | 5.00 |
| Cetearylisononanoat | 6.00 |
| Carbomer | 0.20 |
| Wasser, demin. | ad 100.0 |

**Beispiel 3**

| Hydrodispersionsgel | |
|---|---|
| | Gew.-% |
| Carbomer | 0.50 |
| Butylenglykol | 5.00 |
| Cosmacol ETI | 10.00 |
| Natriumhydroxid (45%ig) | 0.35 |
| Konservierungsmittel | q.s. |
| Parfum | q.s. |
| Uvinul T150 | 5.00 |
| Hydroxypropylcellulose | 0.60 |
| Wasser, demin. | ad 100.00 |

**Patentansprüche**

**1.** Lichtschutzwirksame Wirkstoffkombinationen aus 4,4',4"-(1,3,5-Triazin-2,4,6-triyltri-imino)-tris-benzoesäure-tris(2-ethylhexylester) und einem oder mehreren Dialkylestem von α,β-Dihydroxycarbonsäuren, wobei diese α,β-Dihydroxycarbonsäurediester die allgemeine Formel

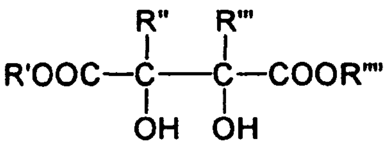

besitzen,

(a) wobei R" und R"'unabhängig voneinander gewählt werden aus der Gruppe

(a1) H- ,
(a2) verzweigtes oder unverzweigtes $C_{1-25}$-Alkyl-,
(a3) mit einer oder mehreren Carboxylgruppen und/oder Hydroxygruppen und/oder Aldehydgruppen und/ oder Oxogruppen (Ketogruppen) substituiertes verzweigtes oder unverzweigtes $C_{1-25}$-Alkyl-

oder
(b) wobei das α-Kohlenstoffatom der α-Hydroxycarbonsäure mit R’ und R" zusammen eine

(b1) unsubstituierte Cycloalkylgruppe mit 3 bis 7 Ringatomen oder eine
(b2) mit einer oder mehreren Carboxylgruppen und/oder Hydroxygruppen und/oder Oxogruppen (Ketogruppen) und/oder verzweigten und/oder unverzweigten $C_{1-25}$-Alkylgruppen substituierte Cycloalkylgruppe mit 3 bis 7 Ringatomen

ausbildet und
wobei R’ und R"" unabhängig voneinander gewählt werden aus der Gruppe verzweigtes oder unverzweigtes $C_{1-25}$-Alkyl-.

**2.** Verwendung von Dialkylestem von α,β-Dihydroxycarbonsäuren, wobei diese α,β-Dihydroxycarbonsäurediester die allgemeine Formel

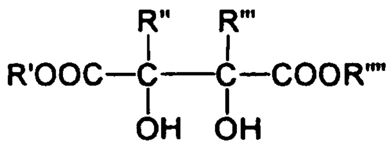

besitzen,

(a) wobei R" und R'"unabhängig voneinander gewählt werden aus der Gruppe

(a1) H- ,
(a2) verzweigtes oder unverzweigtes $C_{1-25}$-Alkyl-,
(a3) mit einer oder mehreren Carboxylgruppen und/oder Hydroxygruppen und/oder Aldehydgruppen und/ oder Oxogruppen (Ketogruppen) substituiertes verzweigtes oder unverzweigtes $C_{1-25}$-Alkyl-

oder
(b) wobei das α-Kohlenstoffatom der α-Hydroxycarbonsäure mit R’ und R" zusammen eine

(b1) unsubstituierte Cycloalkylgruppe mit 3 bis 7 Ringatomen oder eine
(b2) mit einer oder mehreren Carboxylgruppen und/oder Hydroxygruppen und/oder Oxogruppen (Ketogruppen) und/oder verzweigten und/oder unverzweigten $C_{1-25}$-Alkylgruppen substituierte Cycloalkylgruppe mit 3 bis 7 Ringatomen

ausbildet und
wobei R’ und R"" unabhängig voneinander gewählt werden aus der Gruppe verzweigtes oder unverzweigtes $C_{1-25}$-Alkyl-, als Lösungsmittel oder Lösungsvermittler oder Solubilisator für 4,4’,4"-(1,3,5-Triazin-2,4,6-triyltriimino)-tris-benzoesäure-tris(2-ethylhexylester), insbesondere für die Verwendung in Lichtschutzmitteln.

**3.** Kombinationen nach Anspruch 1 oder Verwendung nach Anspruch 2, **dadurch gekennzeichnet, daß**als weitere Lichtschutzfiltersubstanzen Salicylsäurederivate aus der Gruppe (4-Isopropylbenzylsalicylat), (2-Ethylhexylsalicylat, Octylsalicylat) und oder (Homomenthylsalicylat) gewählt werden.

**4.** Kombinationen nach Anspruch 1 oder Verwendung nach Anspruch 2, **dadurch gekennzeichnet, daß** die Gesamtmenge an 4,4’,4"-(1,3,5-Triazin-2,4,6-triyltriimino)-tris-benzoesäure-tris(2-ethylhexylester) in den fertigen kosmetischen oder dermatologischen Zubereitungen aus dem Bereich von 0,1 - 10,0 Gew.-%, bevorzugt 0,5 - 6,0 Gew.-

% gewählt wird, jeweils bezogen auf das Gesamtgewicht der Zubereitungen.

**5.** Kombinationen nach Anspruch 1 oder Verwendung nach Anspruch 2, **dadurch gekennzeichnet, daß** die Gesamtmenge an einem oder mehreren α,β-Dihydroxycarbon-säurediestern in den fertigen kosmetischen oder dermatologischen Zubereitungen aus dem Bereich von 0,1 - 25,0 Gew.-%, bevorzugt 0,5 - 15,0 Gew.-% gewählt wird, jeweils bezogen auf das Gesamtgewicht der Zubereitungen.

**6.** Kombinationen nach Anspruch 1 oder Verwendung nach Anspruch 2, **dadurch gekennzeichnet, daß** Gewichtsverhältnisse von 4,4',4"-(1,3,5-Triazin-2,4,6-triyltriimino)-tris-benzoesäure-tris(2-ethylhexylester) und einem oder mehreren Salicylsäurederivaten aus dem Bereich von 1 : 10 bis 10 : 1, bevorzugt 1 : 4 bis 4 : 1, gewählt werden.

## Claims

**1.** Active compound combinations of tris(2-ethylhexyl) 4,4',4"-(1,3,5-triazine-2,4,6-triyltriimino)-trisbenzoate having sunscreen activity and one or more dialkyl esters of α,β-dihydroxycarboxylic acids, these α,β-dihydroxycarboxylic acid diesters having the general formula

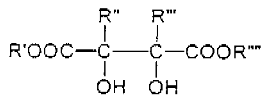

(a) where R" and R"' independently of one another are selected from the group consisting of

(a1) H-,
(a2) branched or unbranched $C_{1-25}$-alkyl-,
(a3) branched or unbranched $C_{1-25}$-alkyl- substituted by one or more carboxyl groups and/or hydroxyl groups and/or aldehyde groups and/or oxo groups (keto groups)

or
(b) where the α-carbon atom of the α,β-dihydroxycarboxylic acid together with R" and R"' forms an

(b1) unsubstituted cycloalkyl group having 3 to 7 ring atoms or a
(b2) cycloalkyl group having 3 to 7 ring atoms and substituted by one or more carboxyl groups and/or hydroxyl groups and/or oxo groups (keto groups) and/or branched and/or unbranched $C_{1-25}$-alkyl groups

and
where R' and R"" independently of one another are selected from the group consisting of branched or unbranched $C_{1-25}$ alkyl-.

**2.** Use of dialkyl esters of α,β-dihydroxycarboxylic acids, these α,β-dihydroxycarboxylic acid diesters having the general formula

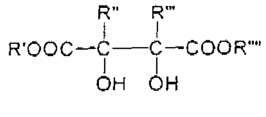

(a) where R" and R"' independently of one another are selected from the group consisting of

(a1) H-,
(a2) branched or unbranched $C_{1-25}$-alkyl-,
(a3) branched or unbranched $C_{1-25}$-alkyl- substituted by one or more carboxyl groups and/or hydroxyl groups and/or aldehyde groups and/or oxo groups (keto groups)

or

(b) where the α-carbon atom of the α,β-dihydroxycarboxylic acid together with R" and R"' forms an

(b1) unsubstituted cycloalkyl group having 3 to 7 ring atoms or a
(b2) cycloalkyl group having 3 to 7 ring atoms and substituted by one or more carboxyl groups and/or hydroxyl groups and/or oxo groups (keto groups) and/or branched and/or unbranched $C_{1-25}$-alkyl groups

and

where R' and R"" independently of one another are selected from the group consisting of branched or unbranched $C_{1-25}$-alkyl-, as solvent or solubilizer for tris(2-ethylhexyl) 4,4',4"-(1,3,5-triazine-2,4,6-triyl-triimino)trisbenzoate, in particular for use in sunscreens.

**3.** Combinations according to Claim 1 or use according to Claim 2, **characterized in that**, as further sunscreen filter substances, salicylic acid derivatives from the group consisting of 4-isopropylbenzyl salicylate, 2-ethylhexyl salicylate or octyl salicylate and homomenthyl salicylate are selected.

**4.** Combinations according to Claim 1 or use according to Claim 2, **characterized in that** the total amount of tris(2-ethylhexyl) 4,4',4"-(1,3,5-triazine-2,4,6-triyl-triimino)trisbenzoate in the finished cosmetic or dermatological preparations is selected from the range 0.1-10.0% by weight, preferably 0.5-6.0% by weight, in each case based on the total weight of the preparations.

**5.** Combinations according to Claim 1 or use according to Claim 2, **characterized in that** the total amount of one or more α,β-dihydroxycarboxylic acid diesters in the finished cosmetic or dermatological preparations is selected from the range 0-1-25.0% by weight, preferably 0.5-15.0% by weight, in each case based on the total weight of the preparations.

**6.** Combinations according to Claim 1 or use according to Claim 2, **characterized in that** weight ratios of tris(2-ethylhexyl) 4,4',4"-(1,3,5-triazine-2,4,6-triyl-triimino)triisbenzoate and one or more salicylic acid derivatives are selected from the range 1:10 to 10:1, preferably 1:4 to 4:1.

## Revendications

**1.** Combinaisons d'ingrédients actifs à activité photoprotectrice, à base d'ester tris(2-éthyl-hexylique) d'acide 4,4', 4"-(1,3,5-triazine-2,4,6-triyltriimino)-tris-benzoïque et d'un ou de plusieurs esters dialkyliques d'acides α,β-dihydroxycarboxyliques, où ces diesters d'acide α,β-dihydroxycarboxylique sont de formule générale :

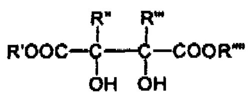

(a) dans laquelle R" et R'" sont choisis, indépendamment l'un de l'autre, parmi le groupe constitué :

(a1) d'un atome d'hydrogène,
(a2) d'un groupe alkyle en $C_{1-25}$ ramifié ou non ramifié,
(a3) d'un groupe alkyle en $C_{1-25}$ ramifié ou non ramifié, substitué par un ou plusieurs groupes carboxyle et/ou groupes hydroxy et/ou groupes aldéhyde et/ou groupes oxo (groupes céto)),

ou

(b) dans laquelle l'atome de carbone en α de l'acide α-hydroxycarboxylique forme, conjointement avec R' et R",

(b1) un groupe cycloalkyle non substitué renfermant de 3 à 7 atomes du noyau, ou
(b2) un groupe cycloalkyle, renfermant de 3 à 7 atomes du noyau, substitué par un

ou plusieurs groupes carboxyle et/ou groupes hydroxy et/ou groupes oxo (groupes céto) et/ou groupes alkyle en $C_{1-25}$ ramifiés et/ou non ramifiés,

et
dans laquelle R' et R'''' sont choisis, indépendamment l'un de l'autre, parmi le groupe constitué d'un groupe alkyle en $C_{1-25}$ ramifié ou non ramifié.

**2.** Utilisation d'esters dialkyliques d'acides α,β-dihydroxycarboxyliques, où ces diesters d'acide α,β-dihydroxycarboxylique sont de formule générale :

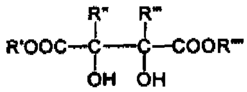

(a) dans laquelle R'' et R''' sont choisis, indépendamment l'un de l'autre, parmi le groupe constitué :

(a1) d'un atome d'hydrogène,
(a2) d'un groupe alkyle en $C_{1-25}$ ramifié ou non ramifié,
(a3) d'un groupe alkyle en $C_{1-25}$ ramifié ou non ramifié, substitué par un ou plusieurs groupes carboxyle et/ou groupes hydroxy et/ou groupes aldéhyde et/ou groupes oxo (groupes céto)),

ou
(b) dans laquelle l'atome de carbone en α de l'acide α-hydroxycarboxylique forme, conjointement avec R' et R'',

(b1) un groupe cycloalkyle non substitué renfermant de 3 à 7 atomes du noyau, ou
(b2) un groupe cycloalkyle, renfermant de 3 à 7 atomes du noyau, substitué par un

ou plusieurs groupes carboxyle et/ou groupes hydroxy et/ou groupes oxo (groupes céto) et/ou groupes alkyle en $C_{1-25}$ ramifiés et/ou non ramifiés,

et
dans laquelle R'' et R'''' sont choisis, indépendamment l'un de l'autre, parmi le groupe constitué d'un groupe alkyle en $C_{1-25}$ ramifié ou non ramifié, en tant que solvant ou promoteur de solubilisation ou agent de solubilisation pour l'ester tris(2-éthyl-hexylique) d'acide 4,4',4''-(1,3,5-triazine-2,4,6-triyltriimino)-tris-benzoïque, en particulier, pour l'utilisation dans des compositions photoprotectrices.

**3.** Combinaisons selon la revendication 1 ou utilisation selon la revendication 2, **caractérisées en ce que** des dérivés de l'acide salicylique, parmi le groupe constitué du salicylate de 4-isopropylbenzyle, du salicylate de 2-éthylhexyle, du salicylate d'octyle et/ou du salicylate d'homomenthyle, sont choisis en tant que substances filtrantes photoprotectrices supplémentaires.

**4.** Combinaisons selon la revendication 1 ou utilisation selon la revendication 2, **caractérisées en ce que** la quantité totale d'ester tris(2-éthylhexylique) d'acide 4,4',4''-(1,3,5-triazine-2,4,6-triyltriimino)-tris-benzoïque dans les préparations cosmétiques ou dermatologiques finies est choisie dans la plage de 0,1 à 10,0 % en poids, de préférence de 0,5 à 6,0 % en poids, dans chaque cas, par rapport au poids total des préparations.

**5.** Combinaisons selon la revendication 1 ou utilisation selon la revendication 2, **caractérisées en ce que** la quantité totale d'un ou de plusieurs diesters d'acide α,β-dihydroxycarboxylique dans les préparations cosmétiques ou dermatologiques finies est choisie dans la plage de 0,1 à 25,0 % en poids, de préférence de 0,5 à 15,0 % en poids, dans chaque cas, par rapport au poids total des préparations.

**6.** Combinaisons selon la revendication 1 ou utilisation selon la revendication 2, **caractérisées en ce que** les rapports pondéraux entre l'ester tris(2-éthylhexylique) d'acide 4,4',4''-(1,3,5-triazine-2,4,6-triyltriimino)-tris-benzoïque et un ou plusieurs dérivés de l'acide salicylique sont choisis dans la plage de 1:10 à 10:1, de préférence, de 1:4 à 4:1.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 685226 A
- DE 3314742 A